# EUROPEAN PATENT APPLICATION

(11) **EP 0 578 322 A2**
(43) Date of publication of application: **12.01.1994**
(21) Application number: 93201967.2
(22) Date of filing: 06.07.1993
(51) Int. Cl.: A61F 2/34

(54) **Acetabulum for an artificial hip joint**

(30) Priority: 07.07.1992 DE 4222218
(71) Applicant: Bristol-Myers Squibb Company, New York, N.Y. 10154 (US)
(72) Inventor: Schug, Martin, D-2406 Stockelsdorf (DE)
(74) Representative: Mays, Julie

(57) **Abstract**

An acetabulum for an artificial hip is disclosed, which has a metal outer shell (2) and an insert piece (20) in the outer shell, which has a recess (22) to accommodate the condyle of the artificial hip on its inner side. In order to accomplish an especially low-wear and shape-stable artificial joint, the insert consists of metal.

## Description

The invention concerns an acetabulum for an artificial hip joint, with a metal outer shell and an insert in the outer shell that has a cavity on its inner side to accommodate a prosthetic hip condyle.

In known acetabula the insert piece may consist of plastic material, which due to heavy strain can become deformed and worn away and cause a disturbance in the natural human environment of the joint and result in inflammation and pain.

Furthermore, there are known acetabula with a metal outer shell and an insert piece of ceramic material. The advantage of insert pieces made of ceramics is that it is easier for a uniform lubricating film to form on this material, thus almost eliminating the formation of abrasive particles. Moreover, deformation of the insert can be avoided, due to the hardness of the material. However, the drawback of ceramic inserts is that the material can fracture under brief, local compressive stresses of high magnitude.

The object of the invention is to modify an acetabulum of the prior art in such a way that the artificial joint is stable in shape, even in the long term, and the ware on the material is reduced.

This object is accomplished in the acetabulum of the invention in that, according to the invention, the insert piece consists of metal.

The advantages of the invention lie particularly in that the use of metal in the interaction with a metal condyle of the prosthetic joint, given very precise fabrication and surface treatment, achieves reduced wear levels, i.e. less abrasion as compared to polyethylene, and thus the formation of particles is reduced in the metal/metal coupling of the artificial joint. Because of the choice of metal, shattering or fracturing of the insert piece is avoided. A major advantage, furthermore, is that familiar and well-tested forms of the outer shell can be used in the invented acetabulum, and it is also possible to use insert pieces of other material as an alternative here. Thus, the surgeon can decide (even during the operation) which insert piece to use.

It is especially preferable that the insert piece be secured in the shell by means of a conical clamp connection, and a particularly preferred embodiment of the invention is to fashion the inner surface of the outer shell as an inner cone, starting at the outer edge of the shell, along a specified axial height, and to configure the outer surface of the insert as an outer cone along the corresponding axial height, both cones having a very small angle and thus generating the necessary clamping action.

According to a preferred embodiment of the invention, the outer shell has a central bore at its pole, and the insert piece has a pin at its pole that protrudes into the borehole, in order to achieve an extra centering of the insert and the outer shell.

To achieve a weight reduction, it is advantageous to fashion the insert piece as a thin-walled shell, so that a cavity is produced between the insert piece and the outer shell. The thin-walled shell-shaped insert in this embodiment has a radially and outwardly directed peripheral flange at its open edge, the circumferential surface of which has sufficient axial height to develop the outer cone on it.

As an alternative, instead of a conical clamp connection the insert piece can also be fastened in the outer shell by a screw connection or separate fastening elements, such as snap rings or the like. In a preferred embodiment of the invention, the outer shell has an open-pore or open-cell surface structure on the outer side, which allows the surrounding bone tissue to grow into it. As an alternative, the outer shell can also be provided with an outer screw thread or other fastening elements that enable a good anchoring in the bone tissue.

According to an especially preferred embodiment of the invention, the inner surface of the shell-shaped insert piece is hard-faced, and the insert consists of a metal, e.g., a cobalt-chromium alloy, that possesses good lubricating properties and an especially reduced wear formation.

Advantageous modifications of the invention are characterized by the features as of the subsidiary claims.

An embodiment of the invention shall now be explained more closely by means of a drawing.

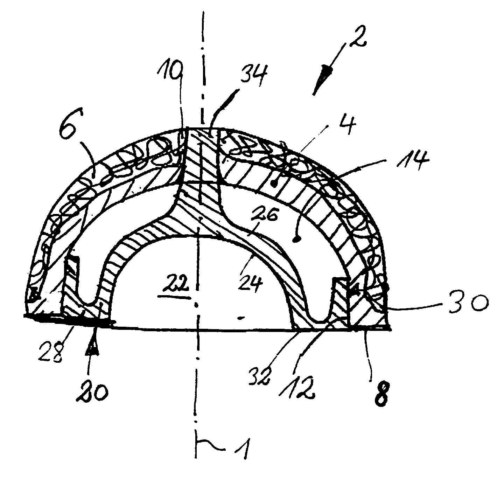

The figure shows an outer shell 2 of metal, which is fashioned in rotational symmetry about an axis of rotation 1, essentially as a hemisphere, and which has an inner wall 4 of solid metal, and on the outside (with respect to the drawing) there is an open-pore or open-cell surface structure 6 into which the surrounding bone tissue can grow, after the implantation. Instead of the open-pore surface structure 6 on the outside there may also be a screw thread or other fastening element to anchor the acetabulum in the natural bone tissue. The outer shell 2 on its open inner side has a circumferential edge 8 that borders on the shell cavity.

A shell-shaped insert piece 20, which has a cavity 22 to accommodate the condyle of an artificial hip at its freely accessible inner side, is placed into the cavity of the outer shell. The cavity 22 has the shape of a spherical segment, and is preferably shaped as a hemisphere and is defined by a polished inner surface 24, which is the bearing surface for the condyle of the artificial hip. The spherically shaped inner surface 24 of the insert piece 20 is located on a shell-shaped, rotationally symmetrical wall 26, which passes into a central pin 34 at its pole, and the pin protrudes into a central bore 10 in the outer shell.

The shell-shaped wall 26 has, at the edge 32 bordering the cavity 22, a radially and outwardly directed flange 28, the circumferential surface of which has sufficient axial height and is fashioned as an outer cone 30. The outer shell 2 has a corresponding inner cone 12 on its inner wall, bordering the circumferential edge 8, with which the outer cone 30 of the insert piece engages to form a conical clamping connection. Between the shell-shaped wall 26 of the insert piece 20 and the outer shell 2, in the embodiment shown, there is a cavity 14 with rotational symmetry. The shell-shaped wall 26 is dimensioned so that the condyle of the artificial hip is securely and reliably accommodated, while on the other hand avoiding too great a wall thickness, which would undesirably increase the weight of the acetabulum. The inner dimension of the outer bowl 2 is designed so that, instead of the metal insert 20, it is also possible to place plastic inserts with substantially larger wall thickness in the outer bowl, so that the insert piece 20 or a plastic insert or even a ceramic insert can be used as alternatives.

## Claims

1. Acetabulum for an artificial hip, with a metal outer shell and an insert piece in the outer shell, having a recess on its inner side to accommodate the condyle of an artificial hip,
**characterised in that** the insert (20) consists of metal.

2. Acetabulum according to claim 1,
**characterised in that** the insert (20) can be secured in the outer shell (2) by means of a conical clamping connection (8, 30).

3. Acetabulum according to claim 2,
**characterised in that** the inner surface (4) of the outer shell (2) is fashioned as an inner cone (12) for a specified axial height, and the outer surface of the insert (20) is fashioned as an outer cone (30) for a corresponding axial height.

4. Acetabulum according to claim 3,
**characterised in that** the inner cone (12) of the outer shell and the outer cone (30) of the insert (20) extend for a specified axial height from the open edge of the shell (8) to the pole of the outer shell.

5. Acetabulum according to one of the previous claims,
**characterised in that** the outer shell (2) has a central bore (10) at its pole, and the insert (20) has a central pin (34) protruding into the bore (10).

6. Acetabulum according to one of the previous claims,
**characterised in that** at least one cavity (14) is provided between the insert (20) and the outer shell (2).

7. Acetabulum according to claim 6,
**characterised in that** the cavity (14) between the insert (20) and the outer shell (2) between the conical clamping connection (12, 30) and the central pin (34) are rotationally symmetrical.

8. Acetabulum according to one of the previous claims,
**characterised in that** the insert (20) is fashioned as a thin-walled inner shell, which has a radially and outwardly directed flange (28) in the edge (27) bordering the recess (22), and the flange is fashioned as an outer cone (30) on its circumferential surface.

9. Acetabulum according to claim 1,
**characterised in that** the outer shell (2) on its inner surface and the insert (20) on its outer surface are provided with interacting threading.

10. Acetabulum according to one of the previous claims,
**characterised in that** the outer shell (2) has an open-pore or open-cell surface structure (6) on its outer surface.
